(19) Europäisches Patentamt
European Patent Office
Office européen des brevets

(11) **EP 2 853 288 B1**

(12) **EUROPÄISCHE PATENTSCHRIFT**

(45) Veröffentlichungstag und Bekanntmachung des Hinweises auf die Patenterteilung:
**30.10.2019 Patentblatt 2019/44**

(51) Int Cl.:
*A61N 1/37* (2006.01)     *A61N 1/05* (2006.01)
*A61N 1/39* (2006.01)     *A61B 5/042* (2006.01)
*A61N 1/365* (2006.01)    *A61N 1/08* (2006.01)

(21) Anmeldenummer: **14174640.4**

(22) Anmeldetag: **27.06.2014**

(54) **Implantierbares Gerät und Herstellungsverfahren für ein implantierbares Gerät**

Implantable device and production method for an implantable device

Appareil implantable et procédé de fabrication d'un appareil implantable

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.08.2013 US 201361869768 P**

(43) Veröffentlichungstag der Anmeldung:
**01.04.2015 Patentblatt 2015/14**

(73) Patentinhaber: **BIOTRONIK SE & Co. KG**
**12359 Berlin (DE)**

(72) Erfinder:
• **Friedrich, Michael**
**14532 Kleinmachnow (DE)**
• **Rump, Jens**
**12049 Berlin (DE)**
• **Büssing, Heinrich**
**10317 Berlin (DE)**

(74) Vertreter: **Galander, Marcus et al**
**Biotronik Corporate Services SE**
**Corporate Intellectual Property**
**Sieversufer 7 - 9**
**12359 Berlin (DE)**

(56) Entgegenhaltungen:
**ES-A1- 2 211 325**     **US-A1- 2008 167 701**
**US-A1- 2011 196 229**     **US-B2- 8 380 322**

**Beschreibung**

Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Die Erfindung betrifft ein permanent oder temporär implantierbares medizinisches Gerät mit einem langgestreckten elektrischen Leiter und ein Herstellungsverfahren für ein implantierbares medizinisches Gerät.

Elektrische Leiter enthaltende medizinische Geräte, beispielsweise Elektrodenleitungen für Elektrostimulation oder Elektroden für Herzschrittmacher, haben den Nachteil, dass sich der elektrische Leiter in einem elektromagnetischen Wechselfeld, zum Beispiel in einem Kernspintomografen, erwärmen kann, weil elektromagnetische Wechselfelder in dem elektrischen Leiter elektrische Ströme induzieren. Die Erwärmung tritt vorzugsweise an Leitungsenden auf und ist abhängig von der Amplitude der Wellen des elektromagnetischen Wechselfeldes, wobei die Erwärmung maximal bei Ausbildung stehender Wellen ist.

Implantierbare Herzschrittmacher oder Defibrillatoren sind typischerweise mit wenigstens einer Stimulationselektrodenleitung verbunden. Der Herzschrittmacher oder Defibrillator enthält an seinem distalen Ende, das vorgesehen ist im Herzen platziert zu werden, einen oder mehrere Elektrodenpole. Solche Elektrodenpole dienen zur Abgabe elektrischer Impulse, beispielsweise an das Gewebe (Myokard) des Herzens oder zum Abfühlen elektrischer Felder, um im Rahmen des sogenannten Sensings eine Aktivität, beispielsweise eine Herzaktivität abfühlen zu können.

Zu diesem Zweck bilden Elektrodenpole typischerweise elektrisch leitende Oberflächenabschnitte einer Elektrodenleitung. Typischerweise sind die Elektrodenpole als Ringelektroden in Form eines Rings um die Elektrodenleitung oder in Form einer Spitzen- oder Tipelektrode am distalen Ende der Elektrodenleitung vorgesehen. Die Elektrodenpole sind über einen oder mehrere elektrische Leiter mit Kontakten eines elektrischen Anschlusses der Elektrodenleitung an deren proximalen Enden elektrisch leitend verbunden. Die elektrischen Leiter können zur Übertragung von Stimulationsimpulsen zu den Elektrodenpolen und/oder zur Übertragung von, von den Elektrodenpolen aufgenommenen, elektrischen Signalen zum proximalen Ende der Elektrodenleitung, genutzt werden. Elektrische Leiter die dem Einsatzzweck der Elektrodenleitung (d.h. der Primär-Funktion der Elektrodenleitung) dienen, werden im Rahmen dieses Textes als Funktionsleiter bezeichnet.

Durch äußere Wechselmagnetfelder können in den Funktionsleitern elektrische Ströme induziert werden, die beispielsweise zu einer Erwärmung der Funktionsleiter und/oder der mit ihnen verbundenen Elektrodenpole führen kann. Falls die Funktionsleiter mit Elektrodenpolen verbunden sind, die im Betrieb Kontakt mit umgebendem Gewebe haben, kann die Induktion von Strömen in einem Funktionsleiter zu einer Erwärmung von an den Funktionsleiter angeschlossenen Elektrodenpolen und zu einer Erwärmung des umgebenden Gewebes führen.

US 7,904,178 B2 zeigt einen langgestreckten Körper einer medizinischen, elektrischen Leitung mit wenigstens einem in eine Spule geformten Leiter. Der Leiter enthält einen ersten sich innerhalb einer Isolationsaußenhülle erstreckenden Abschnitt und einen zweiten Abschnitt der sich außerhalb der Isolationsaußenhülle erstreckt und als Energieabführender Shunt verwendet wird.

US 6,871,091 B2 offenbart eine elektrische Leitung mit einem langgestreckten Körper. Die elektrische Leitung umfasst distale und proximale Endabschnitte, eine erste mit dem distalen Endabschnitt des langgestreckten Körpers verbundene Elektrode und einen ersten sich zwischen distalem und proximalem Endabschnitt erstreckenden Leiter, der elektrisch mit der ersten Elektrode verbunden ist. Eine zweite Elektrode ist mit dem langgestreckten Körper verbunden und ein Kondensator ist elektrisch mit dem ersten Leiter und der zweiten Elektrode verbunden.

ES2211325A1 beschreibt einen langgestreckten, flachen Elektrodenträger für ein Kochliarimplantat, welcher eine Mehrzahl von Elektroden zeigt, die jeweils durch eine Leiterbahn mit einem entsprechenden Kontakt verbunden sind. Die US20080167701A1 beschreibt flexible und/oder dehnbare Elektrodenleitungen zum Einsatz in wechselnden elektromagnetischen und magnetischen Feldern. In der US8380322B2 wird eine Elektrodenleitung beschrieben, die im distalen Bereich vor dem Spitzenkontakt einen Hochfrequenzfilter aufweist. Darüber hinaus beschreibt die US20110196229A1 eine Vorrichtung die in eine Elektrodenleitung eingeführt, elektromagnetische Wechselfelder ausfiltern kann

Der Erfindung liegt die Aufgabe zugrunde, eine mögliche Erwärmung der Elektrodenleitung und/oder des mit der Elektrodenleitung in Kontakt befindlichen Gewebes zu verringern. Ein weiterer Aspekt betrifft ein Verfahren, welches die sichere und kostengünstige Herstellung eines miniaturisierten implantierbaren medizinischen Geräts ermöglicht, das eine verringerte Erwärmung der Elektrodenleitung und/oder des mit der Elektrodenleitung in Kontakt befindlichen Gewebes und eine erhöhte mechanische und elektrische Stabilität aufweist, sowie durch eine hermetische Bauweise bei geringem Einsatz verschiedener Stoffgruppen ausgezeichnet ist.

Erfindungsgemäß wird dies erreicht durch ein temporär oder permanent implantierbares medizinisches Gerät mit wenigstens einer langgestreckten elektrischen Leitung, die einen Funktionsleiter als erste elektrische Komponente oder als Teil einer ersten elektrischen Komponente enthält. Die erste elektrische Komponente des medizinischen Geräts hat wenigstens zu einer zweiten Komponente elektrischen Kontakt, die einen Verbund aus wenigstens einer Metallschicht und wenigstens einer biegeschlaffen Kunststoffschicht aufweist. Dabei ist wenigstens eine der Metallschichten der zweiten Komponente in Serie mit der ersten elektrischen Komponente geschaltet.

Durch eine mit einer Elektrode bzw. der ersten Komponente in Serie geschaltete Metallschicht der zweiten Komponente werden elektromagnetische Wellen im Radiofrequenzbereich zurückreflektiert und somit die Erwärmung des Gewebes insbesondere am oder nahe dem distalen Ende des medizinischen Geräts verringert. Die Einfache und kompakte Bauweise des medizinischen Geräts erzeugt eine hohe mechanische Stabilität, insbesondere in Zugrichtung und vereinfacht die elektrische Kontaktierung zu einer Drahthelix bzw. Innenwendel einer Elektrode. Die Verwendung einer Kunststoffschicht ermöglicht ein hermetisches Versiegeln der leitenden Teile des implantierbaren medizinischen Geräts, wodurch Einflüsse durch elektrolytische Flüssigkeiten nahezu ausgeschlossen werden können und wodurch die Auswahl für die verwendbaren Elemente der Metallschicht erweitert werden kann.

Ein Herstellungsverfahren für das implantierbare medizinische Gerät umfasst, Bereitstellen einer elektrischen Leitung und wenigstens einer zweiten Komponente mit wenigstens einer Metallschicht. Die elektrische Leitung enthält einen Funktionsleiter als erste elektrische Komponente oder als Teil einer ersten elektrischen Komponente. Die Metallschicht der zweiten Komponente wird mit einer biegeschlaffen Kunststoffschicht verbunden. Indem die wenigstens eine Metallschicht der zweiten mit der ersten elektrischen Komponente verbunden wird, wird eine Serienschaltung der ersten mit der zweiten Komponente erzeugt. Bevorzugt werden mehrere zweite Komponenten in Serienschaltung verschaltet.

Durch das Herstellungsverfahren kann ein miniaturisiertes implantierbares medizinisches Gerät hergestellt werden, wobei durch die mit der ersten Komponente in Serie geschaltete Metallschicht der zweiten Komponente elektromagnetische Wellen im Radiofrequenzbereich zurückreflektiert werden und somit die Erwärmung des Gewebes insbesondere am oder nahe dem distalen Ende des medizinischen Geräts verringert wird. Das Herstellungsverfahren erlaubt des Weiteren eine gute Automatisierung bei geringer Anfälligkeit gegenüber von Produktionsintoleranzen, wodurch eine kostengünstige und flexible Serienproduktion ermöglicht werden kann. Das Herstellungsverfahren schließt auch eine sichere elektrische Kontaktierung der Komponenten ein. Dies ist auch bei großer Dimensionierung (> 10 mm) möglich, wobei die implantierbaren medizinischen Geräte durch Teilung miniaturisiert werden können.

In einer bevorzugten Ausgestaltung ist das implantierbare medizinische Gerät eine Elektrodenleitung oder ein Teil einer Elektrodenleitung. Die Elektrodenleitung kann über einen Anschluss mit einem Therapie- oder Monitoringgerät verbunden werden. Das implantierbare medizinische Gerät kann auch ein Teil eines Therapie- oder Monitoringgeräts sein.

In einer besonders bevorzugten Ausgestaltung ist die Breite der Metallschicht der zweiten Komponente geringer als die Breite der Kunststoffschicht, wodurch die zweite Komponente einen elektrisch nichtleitenden Kunststoffrand aufweist. Der Rand kann auf einer oder bevorzugt auf beiden Seiten der zweiten Komponente vorhanden sein. In einer weiteren Ausgestaltung kann der Kunststoffrand einen anderen Kunststoff als die Kunststoffschicht enthalten oder auf die Kunststoffschicht aufgetragen sein. Die Metallschicht der zweiten Komponente kann auch von einer oder mehreren Kunststoffschichten umschlossen sein, beispielsweise in Sandwichbauweise oder dergleichen. Alternativ ist die Metallschicht in eine Kunststoffschicht eingebettet oder auf mehrere Kunststoffschichten aufgetragen.

Bevorzugt ist der Kunststoff ein biegeschlaffer Kunststoff, der sich in, beispielsweise eine Zylinderform oder um einen Zylinder wickeln lässt, ohne dass die zweite Komponente verletzt wird. Die biegeschlaffe Kunststoffschicht der zweiten Komponente kann ein oder mehr Polymere, beispielsweise Polyester, Polyimid, Polyamid, Polytetrafluorethylen, Polypropylen, Polyurethan oder dergleichen enthalten.

Die Metallschicht kann derart ausgebildet sein, dass ein Wickeln in beispielsweise Zylinderform die elektrische Leitung entlang der Metallschicht nicht unterbricht. Die Metallschicht kann ein oder mehrere Metalle, beispielsweise Gold, Silber oder dergleichen enthalten.

In einer weiteren Ausgestaltung ist die Metallschicht der zweiten Komponente mäanderförmig auf die biegeschlaffe Kunststoffschicht aufgetragen. Auch kann die Auftragung der Metallschicht in verschiedenen anderen Formen, beispielsweise zickzackförmig, spiralförmig, labyrinthförmig oder dergleichen erfolgen. Besonders bevorzugt ist die Auftragung der Metallschicht in einer Form, die bei einer Zerteilung der zweiten Komponente entlang einer Achse senkrecht zur Längsachse der flachen zweiten Komponente gleiche, elektrisch entlang einer durchgängigen Metallschicht leitende Teilabschnitte erzeugt, beispielsweise Metallschichtauftragungen mit Mäanderform, Zickzackform oder dergleichen.

Die zweite Komponente kann in zwei oder mehr Teilabschnitte, beispielsweise entlang eines bzw. mehrerer Mäanderarme, bevorzugt an jeweils jedem zweiten Mäanderarm, zerteilt werden. Die Zerteilung der zweiten Komponente kann dabei physikalisch, beispielweise mechanisch, thermisch (d. h. unter Hitzeeinwirkung), wie beispielsweise mittels Laser oder Plasmakauter oder dergleichen und/oder chemisch, beispielsweise mittels Ätzung oder dergleichen erfolgen. Bevorzugt werden die durch die Trennung entstandenen Zylinderflächen der Teilabschnitte durch Druck, Wärme und/oder Lösungsmittel miteinander hermetisch verbunden. Besonders bevorzugt werden dabei die Metallschichten zueinander benachbart angeordneter zweiter Komponenten derart elektrisch miteinander verbunden, dass eine Serienschaltung erzeugt wird.

Die zweite Komponente ist spiralförmig um eine Längsachse aufgewickelt. Es können auch mehrere zweite Komponenten axial entlang einer Längsachse angeordnet um die Längsachse oder koaxial umeinander aufgewickelt sein. Bevorzugt ist die jeweilige wenigstens eine Metallschicht der aufgewickelten zweiten Komponenten elektrisch in der Art mit wenigstens einer Metallschicht einer anderen zweiten Komponente verbunden, so dass eine Serienschaltung erzeugt wird. Die Anzahl der Wicklungen liegt bevorzugt zwischen 50 und 100, besonders bevorzugt zwischen 74 und 76, kann aber auch in Abhängigkeit der Dicke und Länge der zweiten Komponente größer oder kleiner sein. In Abhängigkeit der Form und Wicklung der in den zweiten Komponenten enthaltenen Metall- und Kunststoffschicht können Induktivitäten und dazu parallel geschaltete parasitäre Kapazitäten justiert werden, wodurch ein implantierbares medizinisches Gerät mit Impedanzen von >1 kOhm bei elektrischen Wellen im Radiofrequenzbereich erzeugt werden kann, dass eine geringe Güte auf Grund der Nutzung parasitärer Kapazitäten aufweist, so dass das implantierbare medizinische Gerät über die maximale Impedanz im Resonanzfall hinaus eine signifikant erhöhte Impedanz aufweist und somit breitbandig wirken kann.

Die Längsachse, um die die zweiten Komponenten aufgewickelt werden können, kann zum Beispiel einen Zylinder, einen Dorn, einen Schlauch, ein Kapillarrohr aus Kunststoff oder dergleichen enhalten. Durch das Umwickeln der Längsachse kann eine spiralförmige Leitung bzw. können hohle spiralförmige Leitungsabschnitte erzeugt werden. Die zweite Komponente kann auch die erste Komponente umschließen. Bevorzugt ist die erste Komponente in diesem Fall eine Metallhülse, besonders bevorzugt eine geschlitzte Platinhülse. Eine dritte Komponente, beispielsweise eine weitere Metallhülse, kann die zweite Komponente teilweise oder vollständig umschließen. Auch kann ein Teil der zweiten Komponente aus der dritten Komponente herausragen, falls diese nicht vollständig umschlossen ist. In einer Ausgestaltung ist die dritte Komponente eine geschlitzte Platinhülse mit einem größeren Innendurchmesser als der Außendurchmesser der aufgewickelten zweiten Komponente. Die zweite Komponente kann mit einem Teil aufgewickelt von der Platinhülse umschlossen sein, während ein anderer Teil der zweiten Komponente aus dem Schlitz der Platinhülse herausragt. Die Platinhülse kann eine Elektrode bzw. ein Elektrodenpol zur Abgabe von Stimulationsimpulsen oder zum Erfassen von elektrischen Potentialen sein und mit einer Metallschicht der zweiten Komponente elektrischen Kontakt haben bzw. in Serie verschaltet sein.

Die Dicke der Metallschicht ist bevorzugt geringer als die Breite der Metallschicht, beispielsweise mit einer Dicke zwischen 0,045 $\mu$m und 0,300 $\mu$m und einer Breite von 1,8 mm.

Das Verfahren zur Herstellung eines implantierbaren medizinischen Geräts umfasst bevorzugt einen Schritt, bei dem die Ränder der Kunststoffschicht der zweiten Komponente durch Druck, Wärme und/oder Lösungsmittel miteinander hermetisch verbunden werden.

Des Weiteren kann das Verfahren einen Schritt umfassen, bei dem wenigstens ein Abschnitt der zweiten Komponente physikalisch in axial angeordnete Teilabschnitte zerteilt wird. Die zweite Komponente kann auch chemisch zerteilt werden. Auch können physikalische und chemische Methoden für die Zerteilung gleichzeitig nebeneinander oder chronologisch nacheinander ausgeführt werden, um die Trennung der Abschnitte der zweiten Komponente zu bewirken.

Bevorzugt enthält das Verfahren einen Schritt zum Verbinden von axial angeordneten Teilabschnitten der zweiten Komponente. Die axial angeordneten Teilabschnitte können durch Druck, Wärme und/oder Lösungsmittel miteinander hermetisch verbunden werden. Bevorzugt werden die Metallschichten der axial angeordneten Teilabschnitte derart miteinander verbunden, dass eine Serienschaltung erzeugt wird.

Das implantierbare medizinische Gerät kann eine proximale und eine distale Seite haben. Auch die im implantierbaren medizinischen Gerät enthaltenen Komponenten können proximale und distale Seiten aufweisen. Die distale Seite der ersten elektrischen Komponente hat bevorzugt Kontakt zu einem biologischen Körper, beispielsweise Gewebe, Blut oder dergleichen. Die erste elektrische Komponente kann in diesem Fall Stimulationsimpulse an den biologischen Körper übertragen oder elektrische Signale aus dem Gewebe durch einen am oder nahe dem Ende der ersten Komponente angeordneten Sensor empfangen, um diese elektrischen Signale an das proximale Ende der ersten Komponente zu übertragen. Die Signale können dort von einer Verarbeitungseinheit analysiert bzw. verarbeitet werden, um beispielsweise die Stimulationspulse neu zu justieren.

Die Erfindung soll nun anhand von in den Figuren schematisch abgebildeten Ausführungsbeispielen näher erläutert werden. Von den Figuren zeigen:

Fig. 1   eine schematische Darstellung eines implantierbaren Herzstimulators und eine an diesen angeschlossene implantierbare Elektrodenleitung.

Fig. 2   eine schematische Darstellung eines ersten Ausführungsbeispiels einer Folie eines implantierbaren medizinischen Geräts im unaufgewickelten und im über einen Dorn teilaufgewickelten Zustand.

Fig. 3   eine schematische Darstellung des ersten Ausführungsbeispiels einer Folie eines implantierbaren medizinischen Geräts mit mehr Details.

Fig. 4   eine schematische Darstellung eines ersten Ausführungsbeispiels eines Folienbandstoppfilters.

Fig. 5      eine schematische Darstellung eines zweiten Ausführungsbeispiels einer Folie eines implantierbaren medizinischen Geräts im unaufgewickelten und im über eine Platinhülse teilaufgewickelten Zustand.

Fig. 6      eine schematische Darstellung des zweiten Ausführungsbeispiels einer Folie eines implantierbaren medizinischen Geräts in einem zweiten Ausführungsbeispiel eines Folienbandstoppfilters.

Fig. 7      eine schematische Darstellung des zweiten Ausführungsbeispiels einer Folie eines implantierbaren medizinischen Geräts in einem dritten Ausführungsbeispiel eines Folienbandstoppfilters.

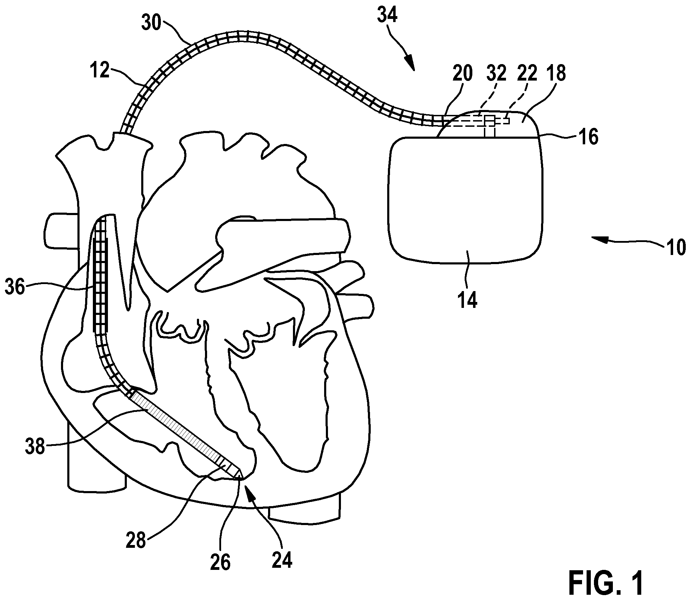

Fig. 1 zeigt als Beispiele implantierbarer medizinischer Geräte einen implantierbaren Herzstimulator 10 und eine mit diesem elektrisch verbundene implantierbare Elektrodenleitung 12.

Der implantierbare Herzstimulator 10 ist im dargestellten Ausführungsbeispiel ein ventrikulärer Herzschrittmacher und Defibrillator, kann aber auch ein Herzschrittmacher, ein Kardioverter/Defibrillator (ICD), ein reines Monitoringgerät zur Herzüberwachung, eine Kombination aus Therapie- und Monitoringgerät, eine Ablationselektrodenleitung oder dergleichen sein.

Der Herzstimulator besitzt ein elektrisch leitendes Metallgehäuse 14, das als großflächiger Elektrodenpol dienen kann. Alternativ kann das Gehäuse 14 auch aus einem anderen, insbesondere nichtleitenden, Material sein. Das Gehäuse 14 hat an seiner Außenseite 16 ein Anschlussgehäuse 18, das auch als Header bezeichnet wird. Das Anschlussgehäuse 18 enthält Kontaktbuchsen 20 mit elektrischen Kontakte 22 zur Aufnahme von Steckkontakten. Die elektrischen Kontakte 22 sind mit Leitern mit einer im Gehäuse 14 des Herzstimulators 10 angeordneten Elektronik verbunden.

Im Folgenden betrachten wir die Elektrodenleitung 12, die ein weiteres implantierbares medizinisches Gerät im Sinne der Erfindung darstellt. Das distale Ende 24 der Elektrodenleitung 12 befindet sich im Apex eines rechten Ventrikels eines Herzens und enthält Elektrodenpole 26 bzw. 28 in Form einer Spitzen- oder Tip-Elektrode 26 und einer in deren Nähe angeordneten Ringelektrode 28. Die Elektrodenpole 26 und 28 sind über einen oder mehrere elektrische Funktionsleiter 30 mit jeweils einem Steckkontakt 32 am proximalen Ende 34 der Elektrodenleitung 12 elektrisch verbunden und können zum Abfühlen elektrischer Potenziale des Herzgewebes (Myokards) oder zur Abgabe elektrische Signale, z. B. in Form von Stimulationspulsen an das sie umgebende Herzgewebe, dienen. Die Funktionsleiter 30 können beispielsweise der Therapie dienende elektrische Signale von dem Steckkontakt 32 zum jeweiligen Elektrodenpol 26 bzw. 28 übertragen oder abgefühlte elektrische Potentiale repräsentierende Signale vom jeweiligen Elektrodenpol 26 bzw. 28 zum Steckkontakt 32 führen und somit der elementaren Funktion der medizinischen Geräte dienen. Die Funktionsleiter 30 sind über einen Großteil ihrer Länge von einer isolierenden Hülle umgeben, so dass ein elektrischer Kontakt zum Gewebe des Herzens gezielt über die Elektrodenpole 26 bzw. 28 zustande kommt. Der Steckkontakt 32 ist mit seinen elektrischen Kontakten mit den elektrischen Kontakten 22 der Kontaktbuchse 20 im Anschlussgehäuse 18 des implantierbaren Herzstimulators 10 verbunden, wodurch Signale von bzw. zu der im Gehäuse 14 des Herzschrittmachers 10 gelegenen Elektronik an die bzw. von den Elektrodenpole(n) 26 bzw. 28 übertragen werden können. Die Elektrodenleitung 12 kann auch Teil der Herzschrittmachers 10 bzw. mit diesem fest verbunden sein.

Die elektrischen Funktionsleiter 30 in der Elektrodenleitung 12 können in unterschiedlichen Längsabschnitten als annähernd gestreckte Seilzugleiter oder als helixförmig gewendelte Leiter bzw. Drahthelices ausgebildet sein. Die Elektrodenleitung 12 im dargestellten Ausführungsbeispiel enthält zusätzlich einen großflächigen proximalen Elektrodenpol 36 und einen großflächigen distalen Elektrodenpol 38, die jeweils von wenigstens einem blank liegenden helixförmig gewendelten Draht gebildet werden und als Defibrillationselektroden 36 und 38 dienen. Die Elektrodenleitung 12 kann auch für die Stimulation und Ableitung von Signalen an Nerven, Gehirn, und anderen Organen oder für die Zuleitung von implantierbaren Sensoren Verwendung finden.

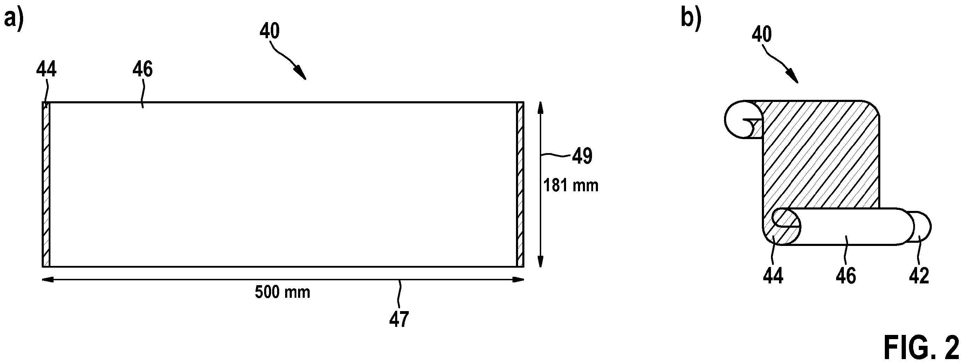

Fig. 2 zeigt ein erstes Ausführungsbeispiel einer Folie 40, die eine zweite Komponente eines implantierbaren medizinischen Geräts darstellt, und die sich in Fig. 2 a) in einem unaufgewickelten Zustand und in Fig. 2 b) in einem über eine Dorn 42 teilaufgewickelten Zustand befindet. Die Folie 40 hat in diesem Ausführungsbeispiel eine Kunststoffschicht 44, beispielsweise aus oder mit einem Polymer wie Polyester, Polyimid, Polyamid, Polytetrafluorethylen, Polypropylen, Polyurethan oder dergleichen und eine auf diese aufgetragene Metallschicht 46, beispielsweise aus Gold oder Silber. Die Metallschicht 46 kann auch aus einem anderen elektrisch leitenden Material sein, beispielsweise Graphen oder dergleichen. Die Kunststoffschicht 44 kann auch aus einem Graphenkomposit oder Fluorographen sein. Die Metallschicht 46 kann auch mit der Kunststoffschicht 44, beispielsweise über Kleben, Pressen oder dergleichen verbunden oder in diese eingebettet sein. In diesem Ausführungsbeispiel hat die Folie 40 eine Länge 47 von 500 mm und eine Breite 49 von 181 mm.

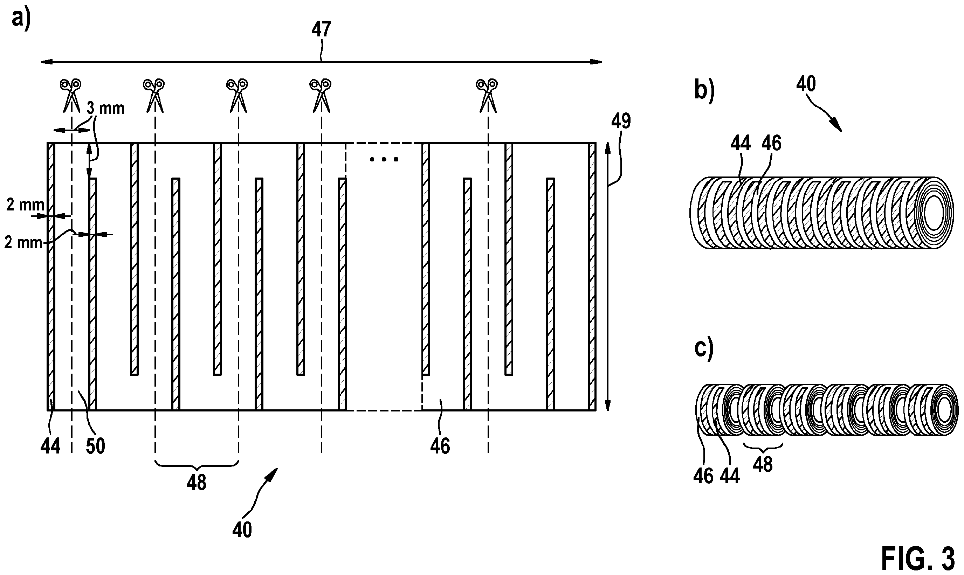

Die Folie 40 kann als Ausgangsmaterial für miniaturisierte implantierbare medizinische Geräte verwendet werden, indem die Folie 40 im unaufgewickelten Zustand wie in Fig. 3 a) gezeigt oder im aufgewickelten Zustand wie in Fig. 3 b) bzw. c) gezeigt, in Teilabschnitte 48 zerteilt wird. Die Zerteilung kann physikalisch, beispielweise mechanisch, thermisch (d. h. unter Hitzeeinwirkung), wie beispielsweise mittels Laser oder Plasmakauter oder dergleichen oder chemisch, beispielsweise mittels Ätzung oder dergleichen erfolgen. Es können auch chronologisch nacheinander oder gleichzeitig mehrere Verfahren zur Zerteilung der Folie 40 in mehrere Teilabschnitte 48 verwendet werden. In diesem Ausführungsbeispiel erfolgt die Zerteilung entlang eines Mäanderarms 50, wodurch kürzere identische Teilabschnitte 48 erzeugt werden. Die Teilabschnitte 48 können durch Druck, Wärme und/oder Lösungsmittel hermetisch miteinander verbunden werden. Auch die Metallschichten 46 der Teilabschnitte 48 können miteinander elektrisch verbunden und somit in Serie geschaltet werden.

Die Metallschicht 46 kann in verschiedenen Formen auf die Kunststoffschicht 44 aufgetragen werden. In diesem Ausführungsbeispiel ist die Metallschicht 46 mäanderförmig aufgetragen, wobei die Breite der mäanderförmigen Metallschicht 3 mm beträgt und diese von Kunststoffschicht-Läufern 44 mit einer Breite von 2 mm umgeben ist. Aufwickeln der Folie 40 erzeugt eine spiralförmige Leitung in Fig. 3 b). Durch Zerteilen, entstehen kürzere spiralförmige Leitungsabschnitte 48 in Fig. 3 c).

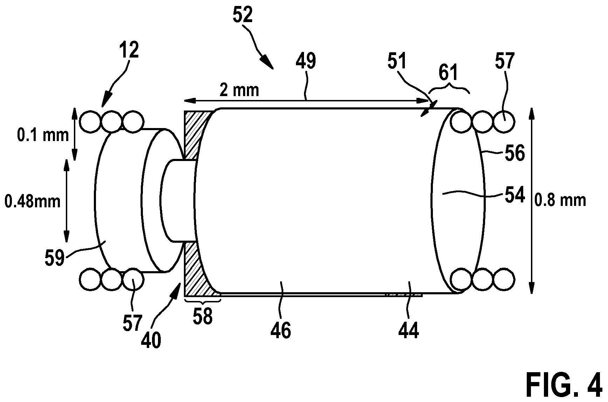

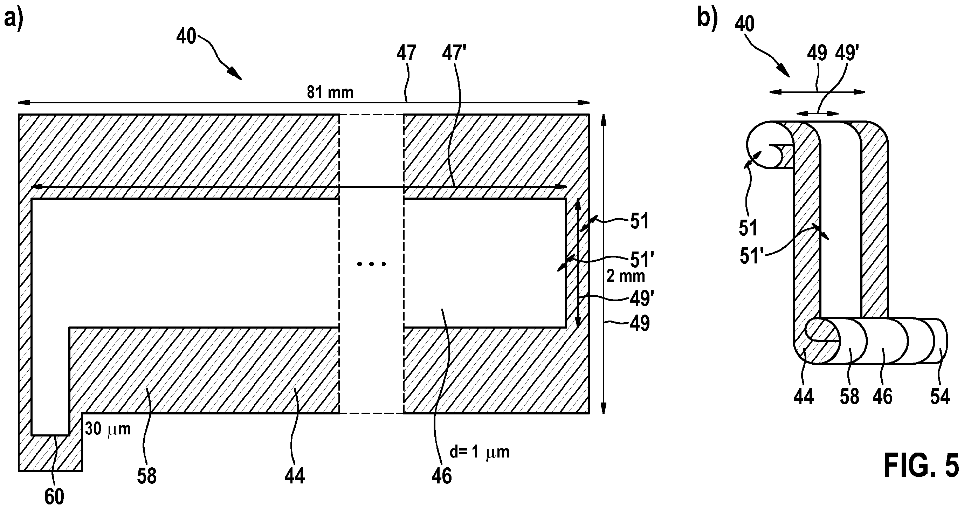

Fig. 4 zeigt ein erstes Ausführungsbeispiel eines Folienbandstoppfilters 52 als weiteres Ausführungsbeispiel für ein implantierbares medizinisches Gerät. Der Folienbandstoppfilter 52 enthält eine innere zylinderförmige mit der Elektrodenleitung 12 elektrisch verbundene Platinhülse 54 um die die Folie 40 spiralförmig aufgewickelt ist. Die innere Platinhülse 54 kann dabei ein Teil eines Funktionsleiters 30 oder mit einem Funktionsleiter 30 der Elektrodenleitung 12 elektrisch verbunden sein. Eine Metallschicht 46 der Folie 40 ist mit der inneren zylinderförmigen Platinhülse 54 elektrisch verbunden und in Serie verschaltet. Die Metallschichten 46 wirken durch das spiralförmige Aufwickeln in 76 Wicklungen als Induktivitäten mit dazu parallel geschalteten parasitären Kapazitäten. Die Anzahl der Wicklungen hängt von der Dicke 51 (s. Fig. 5) der Folie 40 ab, kann auch größer oder kleiner gewählt werden und ist bevorzugt auf den Anwendungsort des implantierbaren medizinischen Geräts angepasst.

Eine zweite äußere zylinderförmige Platinhülse 56 mit einem zum Außendurchmesser der aufgewickelten Folie 40 korrespondierenden Innendurchmesser umschließt die aufgewickelte Folie 40 in diesem Ausführungsbeispiel. Die äußere zylinderförmige Platinhülse 56 kann mit wenigstens einer Metallschicht 46 der Folie 40 elektrisch verbunden und in Serie verschaltet sein. Ein Teil der Folie 40 kann auch durch einen Schlitz in der äußeren Platinhülse 56 nach Außen um die äußere Platinhülse 56 geführt sein (nicht gezeigt). Auch die innere Platinhülse 54 kann geschlitzt sein (nicht gezeigt). Die Hülsen 54 bzw. 56 können auch aus beispielsweise Gold, Silber, Kohlenstoffhanoröhrenkomposit, Kunststoff oder einem anderen leitenden oder nichtleitenden Material sein, bzw. ein solches enthalten. Auch kann nur eine der Hülsen 54 oder 56 ein leitendes Material enthalten, während die andere Hülse 54 oder 56 aus einem nichtleitenden Material sein kann. Die Hülsen 54 oder 56 können auch als Kapillarrohre aus Kunststoff ausgeführt sein, wodurch bei Verwendung des gleichen Materials für die Hülsen 54 und 56 wie für die Kunststoffschicht 44 eine Verbindung der implantierbaren medizinischen Geräte vereinfacht wird. Insbesondere die Verwendung des gleichen Materials für die innere zylinderförmige Hülse 54 und die Kunststoffschicht 44 vereinfacht die mechanische Verbindung der implantierbaren medizinischen Geräte.

Eine elektrische Leitung in Form einer Drahthelix 57 umschließt eine innere Isolationshülle 59 der Elektrodenleitung 12, die eine mit der inneren Platinhülse 54 verbundene innere elektrische Leitung elektrisch von der Drahthelix 57 isoliert. Die innere elektrische Leitung kann auch eine Drahthelix sein (nicht gezeigt).

Die Kunststoffschicht 44 der Folie 40 ist in diesem Ausführungsbeispiel aus Polyester und hat eine Breite 49 von 2 mm, eine Länge 47 von 81 mm, sowie eine Dicke 51 von 1,2 $\mu$m oder 1 $\mu$m (siehe Fig. 5). Die Metallschicht 46 der Folie 40 ist in diesem Ausführungsbeispiel aus Gold und mittig mit einer Breite 49' von 1,8 mm, einer Länge 47' von etwas weniger als 81 mm und einer Dicke 51' von 0,300 $\mu$m auf der Kunststoffschicht 44 angeordnet, sodass auf der Folie 40 ein nichtleitender Kunststoffrand 58 erzeugt wird (siehe Fig. 5). Die Breite 49' der Metallschicht 46 ist grundsätzlich geringer als die Breite 49 der Folie 40 bzw. Kunststoffschicht 44. Alternativ kann die Metallschicht 46 auch innerhalb von zwei Kunststoffschichten 44 in Sandwichbauweise eingebracht sein. Grundsätzlich ist die Dicke 51' der Metallschicht 46 geringer als ihre Breite 49' (siehe Fig. 5). In einem alternativen Ausführungsbeispiel hat die Folie 40 eine Länge von 14 cm (nicht gezeigt).

Die innere Platinhülse 54 ist bevorzugt geschlitzt (nicht gezeigt) und hat einen Innendurchmesser von 0,4 mm, einen Außendurchmesser von 0,48 mm oder 0,5 mm und eine Länge von 2,5 mm. Die Länge der Platinhülse 54 ist somit 0,5 mm größer als die Breite 49 der Folie 40, somit bleibt ein Teil der von der Folie 40 umwickelten Platinhülse 54 frei und kann mit der inneren elektrischen Leitung, beispielsweise einer Drahthelix, einer Elektrode verbunden werden. Die Platinhülse 54 kann auch selbst als Elektrode ausgeführt sein. Die Metallschicht 46 der Folie 40 ist über einen Kontakt 60 (siehe Fig. 5) elektrisch mit der inneren Platinhülse 54 verbunden. Der Kontakt 60 hat in diesem Ausführungsbeispiel eine Breite von 70 $\mu$m und ist vom Außenrand der Folie 40 ca. 30 $\mu$m entfernt. Die Schlitzung der inneren Platinhülse 54 verhindert den Kurzschluss einer Wicklung, der die Induktivität stark herabsetzen würde.

Senkrechtes Abwickeln zur Breite 49 der Folie 40 um die Platinhülse 54 erzeugt einen Außendurchmesser der aufgewickelten Folie 40 von 0,7 mm. Die Folie 40 ist in diesem Ausführungsbeispiel von einer äußeren Platinhülse 56 mit einem Innendurchmesser von 0,68 mm oder 0,7 mm, einem Außendurchmesser von 0,8 mm und einer Länge von 2,5 mm umschlossen, wobei die Folie 40 vollständig bedeckt wird und 5 mm der Folie 40 durch einen Hülsenschlitz (nicht gezeigt) geführt werden. Die Metallschicht 46 der Folie 40 ist mit der Außenhülle der äußeren Platinhülse 56 elektrisch verbunden. Ein Anteil 61 von 0,5 mm der äußeren Platinhülse 56 reicht über die Breite 49 von 2 mm der Folie 40 hinaus, wodurch die äußere Platinhülse mit einer weiterführenden Drahthelix 57 unter der Hülse elektrisch verbunden werden kann. Alternativ kann sich die geschlitzte äußere Platinhülse 54 nach einem zylindrischen Abschnitt von 2 mm, der die Folie 40 umschließt auf einen Außendurchmesser von 0,5 mm verjüngen, so dass die Drahthelix 57 auf der Außenseite der äußeren Platinhülse 56 elektrisch verbunden werden kann.

Ein geringerer Außendurchmesser des implantierbaren medizinischen Geräts kann eine flexible Kontaktierung entlang der gesamten Drahthelix 57 ermöglichen ohne dass weitere bauliche Veränderungen der Elektrode 26 bzw. 28 notwendig sind.

Bevorzugt ist der Folienbandstoppfilter 52 bzw. das implantierbare medizinische Gerät am distalen Ende 24 oder nahe dem distalen Ende 24 der Elektrode 26 angeordnet, um die Erwärmung der Elektrode 26 und des die Elektrode 26 umgebenden Gewebes besonders effektiv zu vermindern. Alternativ oder zusätzlich kann in unmittelbarer Nähe zur Ringelektrode 28, bevorzugt unmittelbar distal zur Ringelektrode 28 ein implantierbares medizinisches Gerät angeordnet sein, wodurch ein zusätzlicher mechanischer Schutz erreicht werden kann.

Fig. 5 a) zeigt ein zweites Ausführungsbeispiel einer Folie 40 mit einer mittig entlang der Länge 47 der Folie 40 verlaufenden Metallschicht 46 mit einer Länge 47', die auf eine Kunststoffschicht 44 aufgetragen ist. Die Metallschicht 46 kann über den Kontakt 60 mit der inneren Platinhülse 54 elektrisch verbunden werden. In Fig. 5 b) ist die Folie 40 auf eine innere Platinhülse 54 teilaufgewickelt und mit dieser elektrisch verbunden. Die Metallschicht 46 auf der Folie 40 hat entlang der Längsachsrichtung der Platinhülse 54 zwei nichtleitende Kunststoffränder 58.

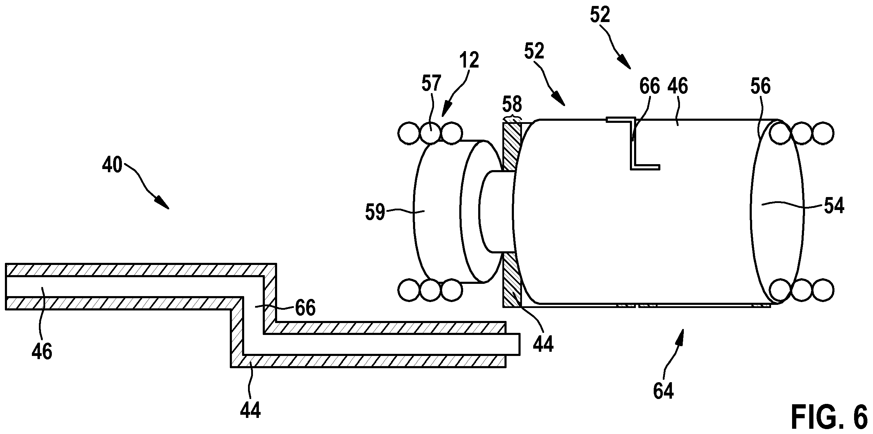

Fig. 6 zeigt das zweiten Ausführungsbeispiels einer Folie 40 in einem zweiten Ausführungsbeispiel eines Folienbandstoppfilters 52. In diesem Ausführungsbeispiel umschließen zwei spiralförmig gewickelte Folien 40 eine innere Platinhülse 54 und sind selbst von einer äußeren Platinhülse 56 umschlossen. Eine proximale Folie 62 ist mit der Außenwand der inneren Platinhülse 54 elektrisch verbunden. Eine zweite, distale Folie 64 ist an ihrem proximalen Ende mit der proximalen Folie 62 und an ihrem distalen Ende mit der äußeren Platinhülse 56 elektrisch verbunden, wodurch eine Serienschaltung der Platinhülsen 54 und 56 mit den Folien 62 und 64 realisiert ist. Das serielle Verschalten mehrerer Folien 40, die als Filter dienen, ermöglicht einen erwünschten Frequenzgang zu erreichen. Die Serienschaltung ist dabei durch treppenförmig zugeschnittene Folien 40 realisiert, wobei nach jeder gewickelten Folie 40 ein elektrisch leitender Steg 66 von der Metallschicht 46 der Folie 62 in die Nähe der Außenwand der inneren Platinhülse 54 zurückgeführt wird um diesen mit einer Metallschicht 46 der axial benachbarten Folie 64 elektrisch zu verbinden. Hierbei ist jedoch die auf die erste Folie 62 folgende Folie 64 nicht mit der inneren Platinhülse 54 elektrisch verbunden, da dies zu einer Parallelschaltung führen würde. Zylinderartige Flächen der axial benachbarten Folien 62 und 64, können durch Druck, Wärme und/oder Lösungsmittel hermetisch miteinander verbunden werden, um den Folienbandstoppfilter 52 hermetisch zu verschließen.

Alternativ kann die distale Folie 64 auch mit der inneren Platinhülse 54 elektrisch verbunden sein, wodurch die Bandbreite des implantierbaren medizinischen Geräts angepasst werden kann. Durch die Konfigurationsmöglichkeiten des Folienbandstoppfilters 52 können sowohl Bandbreite als auch Durchlassfrequenzen so eingestellt werden, dass ein Abkühlungseffekt auch in mehreren Frequenzbändern auftreten und die Fertigungstoleranz erhöht werden kann.

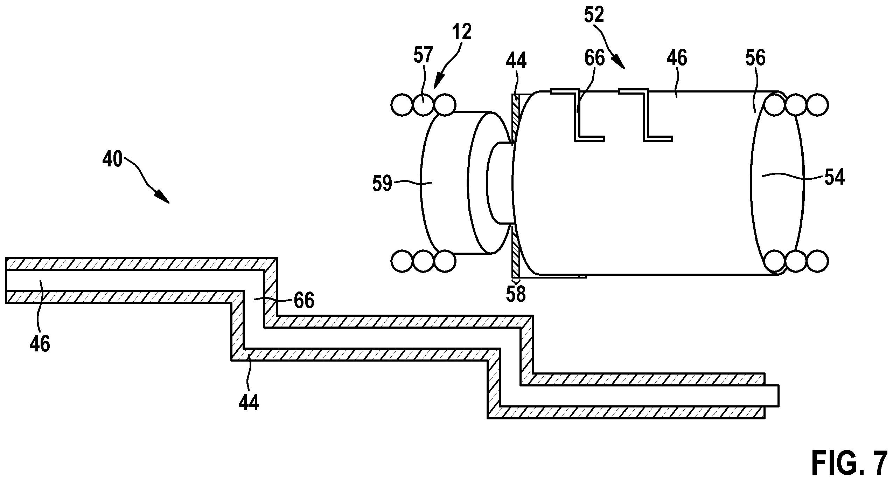

Fig. 7 zeigt das zweiten Ausführungsbeispiels der Folie 40 in einem dritten Ausführungsbeispiel eines Folienbandstoppfilters 52. Der Folienbandstoppfilter 52 in diesem Ausführungsbeispiel ist weitgehend identisch zu dem Folienbandstoppfilter 52 aus dem zweiten Ausführungsbeispiel in Fig. 6, umfasst jedoch drei anstatt zwei in Serie geschalteter Folienabschnitte.

Die genauen geometrischen Abmessungen des biegeschlaffen Folienbandstoppfilters 52 richten sich nach den äußeren Restriktionen des Einsatzortes (maximaler Außendurchmesser $r_2$, minimaler Innendurchmesser $r_1$ und maxi-male Länge b), der Materialwahl der Kunststoffschicht 44 bzw. des Foliensubstrates und der Form und des Materials der Metallschicht 46 bzw. Metallisierung sowie der gewünschten Resonanzfrequenz.

Im Folgenden wird ein Folienbandstoppfilter 52 ähnlich zu dem in Fig. 7 dargestellten angenommen, der jedoch aus Teilabschnitten 48 der Folie 40 aus Fig. 2 bzw. Fig. 3 aufgebaut ist, die eine mäanderförmige Metallschicht 46 aufweisen. Die Resonanzfrequenz eines solchen Folienbandstoppfilters 52 ergibt sich aus:

$$f = \frac{1}{2\pi}\sqrt{\frac{1}{LC} - \frac{R^2}{L^2}}$$

mit der Induktivität L und der Kapazität C, die sich aus den parasitären Kapazitäten zwischen den Spiralarmen abschätzen lässt sowie dem Widerstand R des Folienbandstoppfilters 52. Die Kapazität lässt sich in erster Näherung aus der Kapazität in Reihe geschalteter Tonnenkondensatoren berechnen, dabei gilt:

$$\frac{1}{C} = \sum_n \frac{1}{C_n} \text{ mit } C_n = 2\pi\varepsilon_0\varepsilon_r \frac{b}{\lg\left(\dfrac{r_1 + (n-1)d}{r_1 + nd}\right)}$$

mit Dielektrizitätskonstante $\varepsilon_0$, relativer Permittivität $\varepsilon_r$ der Kunststoffschicht 44, der Breite b des Mäanderarms 50 der Metallschicht 46, der Dicke d der Folie 40 und der Anzahl der Spiralwicklungen n der Folie 40.

Die Induktivität des spiralförmigen Folienbandstoppfilters 52 lässt sich mittels folgender Formel abschätzen (

$$r = \left(\frac{r_1 + r_2}{2}\right)$$

in cm, mit $r_1$ dem Innendurchmesser und $r_2$ dem Außendurchmesser):

$$L = r^2 n^2 / 20r + 28(r_2 - r_1)$$

Ein weiteres Ausführungsbeispiel ist für den Einbau in eine Schrittmacherelektrode für Frequenzen um 64 MHz optimiert und umfasst die folgenden Parameter: Eine Kunststoffschicht 44 in Form einer Polymerfolie aus Polyester mit einer Dicke 51 von d = 1.855 $\mu$m mit einer relativen Permittivität von $\varepsilon_r \sim 3$ und eine Metallschicht 46 aus Silber mit einer Dicke 51' von d = 0.045 $\mu$m, wobei die Folie 40 einen Außendurchmesser von $r_2$ = 780 $\mu$m, einen Innendurchmesser von r1 = 500 $\mu$m, eine Länge 47 von b = 3 mm und 74 Wicklungen umfasst.

Bezugszeichenliste

| | |
|---|---|
| 10 | Herzschrittmacher |
| 12 | Elektrodenleitung |
| 14 | Gehäuse des Herzschrittmachers |
| 16 | Außenseite des Herzschrittmachers |
| 18 | Anschlussgehäuse |
| 20 | Kontaktbuchsen |
| 22 | elektrische Kontakte des Herzschrittmachers |
| 24 | distales Ende der Elektrodenleitung |
| 26 | Spitzen-/Tip-Elektrode |
| 28 | Ringelektrode |
| 30 | Funktionsleiter |
| 32 | Steckkontakt der Elektrodenleitung |
| 34 | proximales Ende der Elektrodenleitung |
| 36 | großflächiger proximaler Elektrodenpol |
| 38 | großflächiger distaler Elektrodenpol |
| 40 | Folie |
| 42 | Dorn |
| 44 | Kunststoffschicht |
| 46 | Metallschicht |
| 47 | Länge |
| 48 | Teilabschnitt |
| 49 | Breite |
| 50 | Mäanderarm |
| 51 | Dicke |
| 52 | Folienbandstoppfilter |
| 54 | innere zylinderförmige Platinhülse |
| 56 | äußere zylinderförmige Platinhülse |
| 57 | Drahthelix |
| 58 | Kunststoffrand der Folie |
| 59 | innere Isolationshülle |
| 60 | Kontakt auf der Metallschicht |

61 Teil der äußeren Platinhülse
62 proximale Folie
64 distale Folie
66 elektrisch leitender Steg

**Patentansprüche**

1. Temporär oder permanent implantierbares medizinisches Gerät (10, 12; 52), welches wenigstens eine langgestreckte elektrische Leitung (12) aufweist, die einen Funktionsleiter (30; 54, 56, 57) als erste elektrische Komponente (30; 54, 57) oder als Teil einer ersten elektrischen Komponente (30; 54, 57) enthält, wobei die erste elektrische Komponente (30; 54, 57) wenigstens mit einer einen Verbund aus wenigstens einer Metallschicht (46) und wenigstens einer biegeschlaffen Kunststoffschicht (44) aufweisenden zweiten Komponente (40; 62, 64) elektrischen Kontakt hat, wobei wenigstens eine der Metallschichten (46) der zweiten Komponente (40; 62, 64) in Serie mit der ersten elektrischen Komponente (30; 54, 57) geschaltet ist, **dadurch gekennzeichnet, dass** die zweite Komponente (40; 62, 64) spiralförmig um die erste Komponente (30; 54) aufgewickelt ist.

2. Implantierbares medizinisches Gerät (10, 12; 52) nach Anspruch 1, wobei das implantierbare medizinische Gerät (10, 12; 52) eine Elektrodenleitung (12) oder Teil einer Elektrodenleitung (12) zum Anschluss oder als Teil eines Therapie- und/oder Monitoringgeräts (10, 12, 26, 28) ist.

3. Implantierbares medizinisches Gerät (10, 12; 52) nach Anspruch 1 und/oder 2, wobei eine Breite (49') der wenigstens einen Metallschicht (46) der zweiten Komponente (40; 62, 64) geringer ist als die Breite (49) der wenigstens einen Kunststoffschicht (44), wodurch die zweite Komponente (40; 62, 64) zwei laterale Kunststoffschicht aufweisende Ränder (58) aufweist.

4. Implantierbares medizinisches Gerät (10, 12; 52) nach wenigstens einem der Ansprüche 1 bis 3, wobei die wenigstens eine Metallschicht (46) der zweiten Komponente (40; 62, 64) mäanderförmig auf die biegeschlaffe Kunststoffschicht (44) aufgetragen ist.

5. Implantierbares medizinisches Gerät (10, 12; 52) nach Anspruch 4, wobei die zweite Komponente (40; 62, 64) entlang eines Mäanderarms (50) in wenigstens zwei Teilabschnitte (48) geteilt ist, wodurch wenigstens zwei getrennte zweite Komponenten (62, 64) mit wenigstens zwei getrennten Metallschichten (46) entstehen und wobei wenigstens eine Metallschicht (46) eines Teilabschnitts (48) zu wenigstens einer Metallschicht (46) eines anderen Teilabschnitts (48) in Serie geschaltet ist.

6. Implantierbares medizinisches Gerät (10, 12; 52) nach wenigstens einem der Ansprüche 1 bis 5, wobei die erste Komponente (30; 54, 57) von der einen Verbund aus wenigstens einer Metallschicht (46) und wenigstens einer biegeschlaffen Kunststoffschicht (44) aufweisenden zweiten Komponente (40; 62, 64) umschlossen ist, und wobei die zweite Komponente (40; 62, 64) von einer dritten elektrischen Komponente (56) umschlossen ist, die mit wenigstens einer Metallschicht (46) der zweiten Komponente (40; 62, 64) elektrisch verbunden ist.

7. Implantierbares medizinisches Gerät (10, 12; 52) nach wenigstens einem der Ansprüche 1 bis 6, wobei eine Dicke (51') der Metallschicht (46) geringer ist als eine Breite (49') der Metallschicht (46).

8. Implantierbares medizinisches Gerät (10, 12; 52) nach wenigstens einem der Ansprüche 1 bis 7, wobei die Kunststoffschicht (44) wenigstens ein Polymer aufweist.

9. Verfahren zur Herstellung eines implantierbaren medizinischen Geräts (10, 12; 52) nach wenigstens einem der Ansprüche 1 bis 8, wobei das Verfahren umfasst:

   - Bereitstellen einer elektrischen Leitung (12), die einen Funktionsleiter (30; 54, 56, 57) als erste elektrische Komponente (30; 54, 57) oder als Teil einer ersten elektrischen Komponente (30; 54, 57) aufweist,
   - Bereitstellen wenigstens einer zweiten Komponente (40; 62, 64) mit wenigstens einer Metallschicht (46) und wenigstens einer mit der wenigstens einen Metallschicht (46) verbundenen biegeschlaffen Kunststoffschicht (44) und
   - elektrisches Verbinden der wenigstens einen Metallschicht (46) der zweiten (40; 62, 64) mit der ersten elektrischen Komponente (30; 54, 57),

wobei die Metallschicht (46) der zweiten Komponente (40; 62, 64) in Serie mit der ersten elektrischen Komponente (30; 54, 57) geschaltet wird.

10. Verfahren zur Herstellung eines implantierbaren medizinischen Geräts (10, 12; 52) nach Anspruch 9, wobei Ränder (58) der Kunststoffschicht (44) der zweiten Komponente (40; 62, 64) durch Druck, Wärme und/oder Lösungsmittel hermetisch miteinander verbunden werden.

11. Verfahren zur Herstellung eines implantierbaren medizinischen Geräts (10, 12; 52) nach Anspruch 9 und/oder 10, wobei wenigstens ein Abschnitt der zweiten Komponente (40; 62, 64) physikalisch in axial angeordnete Teilabschnitte (48) zerteilt wird.

12. Verfahren zur Herstellung eines implantierbaren medizinischen Geräts (10, 12; 52) nach wenigstens einem der Ansprüche 9 bis 11, wobei wenigstens ein Abschnitt der zweiten Komponente (40; 62, 64) chemisch in axial angeordnete Teilabschnitte (48) zerteilt wird.

13. Verfahren zur Herstellung eines implantierbaren medizinischen Geräts (10, 12; 52) nach Anspruch 11 und/oder 12, wobei zylinderartige Flächen der axial angeordneten Teilabschnitte (48) der zweiten Komponente (40; 62, 64) durch Druck, Wärme und/oder Lösungsmittel hermetisch miteinander verbunden werden.

14. Verfahren zur Herstellung eines implantierbaren medizinischen Geräts (10, 12; 52) nach wenigstens einem der Ansprüche 11 bis 13, wobei wenigstens jeweils eine Metallschicht (46) eines jeweiligen axial angeordneten Teilabschnitts (48) und eine benachbart angeordnete Metallschicht (46) eines jeweiligen axial angeordneten Teilabschnitts (46) in einer Serienschaltung elektrisch leitend miteinander verbunden werden.

## Claims

1. A temporarily or permanently implantable medical device (10, 12; 52), comprising at least one elongate electrical line (12) comprising a functional conductor (30; 54, 56, 57) as a first electrical component (30; 54, 57) or as part of a first electrical component (30; 54, 57),
the first electrical component (30; 54, 57) being at least in electrical contact with a second component (40; 62, 64) comprising a composite composed of at least one metal layer (46) and at least one flexible plastic layer (44), at least one of the metal layers (46) of the second component (40; 62, 64) being connected in series to the first electrical component (30; 54, 57), **characterized in that** the second component (40; 62, 64) is wound in a spiral manner about the first electrical component (30; 54).

2. The implantable medical device (10, 12; 52) according to claim 1, wherein the implantable medical device (10, 12; 52) is an electrode lead (12) or part of an electrode lead (12) to connect to or as part of a therapy and/or monitoring device (10, 12, 26, 28).

3. The implantable medical device (10, 12; 52) according to claim 1 and/or 2, wherein a width (49') of the at least one metal layer (46) of the second component (40; 62, 64) is less than the width (49) of the at least one plastic layer (44), whereby the second component (40; 62, 64) has two lateral edges (58) comprising a plastic layer.

4. The implantable medical device (10, 12; 52) according to at least one of claims 1 to 3, wherein the at least one metal layer (46) of the second component (40; 62, 64) is applied to the flexible plastic layer (44) in a meander-shaped manner.

5. The implantable medical device (10, 12; 52) according to claim 4, wherein the second component (40; 62, 64) is divided along a meander arm (50) into at least two subsections (48), whereby at least two separate second components (62, 64) comprising at least two separate metal layers (46) are created, and at least one metal layer (46) of a subsection (48) is connected in series to at least one metal layer (46) of another subsection (48).

6. The implantable medical device (10, 12; 52) according to at least one of claims 1 to 5, wherein the first component (30; 54, 57) is enclosed by the second component (40; 62, 64) comprising a composite composed of at least one metal layer (46) and at least one flexible plastic layer (44), and the second component (40; 62, 64) is enclosed by a third electrical component (56), which is electrically connected to at least one metal layer (46) of the second component (40; 62, 64).

7.  The implantable medical device (10, 12; 52) according to at least one of claims 1 to 6, wherein a thickness (51') of the metal layer (46) is less than a width (49') of the metal layer (46).

8.  The implantable medical device (10, 12; 52) according to at least one of claims 1 to 7, wherein the plastic layer (44) comprises at least one polymer.

9.  A method for producing an implantable medical device (10, 12; 52) according to at least one of claims 1 to 8, the method comprising:

- providing an electrical line (12) comprising a functional conductor (30; 54, 56, 57) as a first electrical component (30; 54, 57) or as part of a first electrical component (30; 54, 57);
- providing at least one second component (40; 62, 64) comprising at least one metal layer (46) and at least one flexible plastic layer (44) connected to the at least one metal layer (46); and
- electrically connecting the at least one metal layer (46) of the second component (40; 62, 64) to the first electrical component (30; 54, 57),

the metal layer (46) of the second component (40; 62, 64) being connected in series to the first electrical component (30; 54, 57).

10. The method for producing an implantable medical device (10, 12; 52) according to claim 9, wherein edges (58) of the plastic layer (44) of the second component (40; 62, 64) are hermetically interconnected by pressure, heat and/or solvent.

11. The method for producing an implantable medical device (10, 12; 52) according to claims 9 and/or 10, wherein at least one section of the second component (40; 62, 64) is physically divided into axially arranged subsections (48).

12. The method for producing an implantable medical device (10, 12; 52) according to at least one of claims 9 to 11, wherein at least one section of the second component (40; 62, 64) is chemically divided into axially arranged subsections (48).

13. The method for producing an implantable medical device (10, 12; 52) according to claims 11 and/or 12, wherein cylindrical faces of the axially arranged subsections (48) of the second component (40; 62, 64) are hermetically interconnected by pressure, heat and/or solvent.

14. The method for producing an implantable medical device (10, 12; 52) according to at least one of claims 11 to 13, wherein at least one respective metal layer (46) of a respective axially arranged subsection (48) and an adjacently arranged metal layer (46) of a respective axially arranged subsection (46) are electrically conductively interconnected in a series connection.

## Revendications

1.  Appareil médical implantable (10, 12 ; 52) de manière temporaire ou permanente, lequel présente au moins une ligne électrique (12) allongée qui contient un conducteur fonctionnel (30 ; 54, 56, 57) en tant que premier composant (30 ; 54, 57) électrique ou en tant que partie d'un premier composant (30 ; 54, 57) électrique, où le premier composant (30 ; 54, 57) électrique a un contact électrique au moins avec un deuxième composant (40 ; 62, 64) présentant un composite à base d'au moins une couche métallique (46) et d'au moins une couche de matière plastique (44) souple en flexion, où au moins une des couches métalliques (46) du deuxième composant (40 ; 62, 64) est connectée en série avec le premier composant (30 ; 54, 57) électrique, **caractérisé en ce que** le deuxième composant (40 ; 62, 64) est enroulé en forme de spirale autour du premier composant (30 ; 54).

2.  Appareil médical implantable (10, 12 ; 52) selon la revendication 1, où l'appareil médical implantable (10, 12 ; 52) est une ligne d'électrode (12) ou une partie d'une ligne d'électrode (12) pour un raccordement, ou est sous forme de partie d'un appareil thérapeutique et/ou de surveillance (10, 12, 26, 28).

3.  Appareil médical implantable (10, 12 ; 52) selon la revendication 1 et/ou la revendication 2, dans lequel la largeur (49') de l'au moins une couche métallique (46) du deuxième composant (40 ; 62, 64) est inférieure à la largeur (49) de l'au moins une couche de matière plastique (44), ce par quoi le deuxième composant (40 ; 62, 64) présente deux bandes (58) latérales présentant une couche de matière plastique.

**4.** Appareil médical implantable (10, 12 ; 52) selon au moins l'une des revendications 1 à 3, dans lequel l'au moins une couche métallique (46) du deuxième composant (40 ; 62, 64) est appliquée sous forme de méandres sur la couche de matière plastique (44) souple en flexion.

**5.** Appareil médical implantable (10, 12 ; 52) selon la revendication 4, dans lequel le deuxième composant (40 ; 62, 64) est divisé en au moins deux segments partiels (48) le long d'un bras de méandre (50), ce par quoi au moins deux deuxièmes composants (62, 64) séparés sont créés avec au moins deux couches métalliques (46) séparées et où au moins une couche métallique (46) d'un segment partiel (48) est connectée en série avec au moins une couche métallique (46) d'un autre segment partiel (48).

**6.** Appareil médical implantable (10, 12 ; 52) selon au moins l'une des revendications 1 à 5, dans lequel le premier composant (30 ; 54, 57) est entouré par le deuxième composant (40 ; 62, 64) présentant un composite à base d'au moins une couche métallique (46) et d'au moins une couche de matière plastique (44) souple en flexion, et dans lequel le deuxième composant (40 ; 62, 64) est entouré par un troisième composant (56) électrique qui est relié électriquement avec au moins une couche métallique (46) du deuxième composant (40 ; 62, 64).

**7.** Appareil médical implantable (10, 12 ; 52) selon au moins l'une des revendications 1 à 6, dans lequel l'épaisseur (51') de la couche métallique (46) est inférieure à une largeur (49') de la couche métallique (46).

**8.** Appareil médical implantable (10, 12 ; 52) selon au moins une des revendications 1 à 7, dans lequel la couche de matière plastique (44) présente au moins un polymère.

**9.** Procédé de fabrication d'un appareil médical implantable (10, 12 ; 52) selon au moins l'une des revendications 1 à 8, où le procédé comprend :

- la mise en place d'une ligne électrique (12), qui présente un conducteur fonctionnel (30 ; 54, 56, 57) en tant que premier composant (30 ; 54, 57) électrique ou en tant que partie d'un premier composant (30 ; 54, 57) électrique,
- la mise en place d'au moins un deuxième composant (40 ; 62, 64) doté d'au moins une couche métallique (46) et doté d'une couche de matière plastique (44) souple en flexion reliée avec l'au moins une couche métallique (46), et
- la connexion électrique de l'au moins une couche métallique (46) du deuxième composant (40 ; 62, 64) avec le premier composant (30 ; 54, 57) électrique,

où la couche métallique (46) du deuxième composant (40 ; 62, 64) est connectée en série avec le premier composant (30 ; 54, 57) électrique.

**10.** Procédé de fabrication d'un appareil médical implantable (10, 12 ; 52) selon la revendication 9, dans lequel des bords (58) de la couche de matière plastique (44) du deuxième composant (40 ; 62, 64) sont reliés hermétiquement ensemble par la pression, la chaleur et/ou un solvant.

**11.** Procédé de fabrication d'un appareil médical implantable (10, 12; 52) selon la revendication 9 et/ou la revendication 10, dans lequel au moins un segment du deuxième composant (40 ; 62, 64) est divisé physiquement en segments partiels (48) disposés axialement.

**12.** Procédé de fabrication d'un appareil médical implantable (10, 12 ; 52) selon au moins l'une des revendications 9 à 11, dans lequel au moins un segment du deuxième composant (40 ; 62, 64) est divisé par voie chimique en segments partiels disposés axialement.

**13.** Procédé de fabrication d'un appareil médical implantable (10, 12 ; 52) selon la revendication 11 et/ou la revendication 12, dans lequel des surfaces de type cylindrique des segments partiels (48) du deuxième composant (40 ; 62, 64) disposés axialement sont reliés hermétiquement ensemble par la pression, la chaleur et/ou un solvant.

**14.** Procédé de fabrication d'un appareil médical implantable (10, 12 ; 52) selon au moins l'une des revendications 11 à 13, dans lequel respectivement une couche métallique (46) d'un segment partiel (48) disposé axialement et une couche métallique (46) disposée au voisinage d'un segment partiel (46) respectif disposé axialement sont reliées ensemble de manière conductrice électriquement dans un circuit en série.

FIG. 1

EP 2 853 288 B1

a)

44    46    40

49
181 mm

500 mm

47

b)

40

44   46   42

EP 2 853 288 B1

FIG. 2

FIG. 3

**FIG. 4**

EP 2 853 288 B1

a)

40

81 mm

47  47'

51

51'

2 mm

49'

49

30 µm

60   58   44   46   d= 1 µm

b)   40

49  49'

51
51'

44  58  46  54

FIG. 5

EP 2 853 288 B1

18

FIG. 6

FIG. 7

**IN DER BESCHREIBUNG AUFGEFÜHRTE DOKUMENTE**

**In der Beschreibung aufgeführte Patentdokumente**

- US 7904178 B2
- US 6871091 B2
- ES 2211325 A1

- US 20080167701 A1
- US 8380322 B2
- US 20110196229 A1